**Europäisches Patentamt**

**European Patent Office**

**Office européen des brevets**

(11) Numéro de publication : **0 365 370 B1**

(12)    **FASCICULE DE BREVET EUROPEEN**

(45) Date de publication du fascicule du brevet :
07.10.92 Bulletin 92/41

(51) Int. Cl.$^5$ : **A61K 7/42**

(21) Numéro de dépôt : **89402310.0**

(22) Date de dépôt : **21.08.89**

(54) **Composition cosmétique filtrante photostable sous forme d'émulsion contenant un filtre hydrosoluble à large bande d'absorption et au moins un filtre UV-A liposoluble.**

Le dossier contient des informations techniques présentées postérieurement au dépôt de la demande et ne figurant pas dans le présent fascicule.

(30) Priorité : **24.08.88 FR 8811178**

(43) Date de publication de la demande :
**25.04.90 Bulletin 90/17**

(45) Mention de la délivrance du brevet :
**07.10.92 Bulletin 92/41**

(84) Etats contractants désignés :
**AT BE CH DE ES FR GB GR IT LI NL SE**

(56) Documents cités :
**GB-A- 2 121 801**
**GB-A- 2 123 418**
**GB-A- 2 133 985**
**GB-A- 2 170 105**
**GB-A- 2 198 944**
**GB-A- 2 200 552**

(73) Titulaire : **L'OREAL**
**14, Rue Royale**
**F-75008 Paris (FR)**

(72) Inventeur : **Grollier, Jean-François**
**16bis Boulevard Morland**
**F-75004 Paris (FR)**

(74) Mandataire : **Casalonga, Axel et al**
**BUREAU D.A. CASALONGA - JOSSE**
**Morassistrasse 8**
**W-8000 München 5 (DE)**

Jouve, 18, rue Saint-Denis, 75001 PARIS

## Description

La présente invention est relative à une composition cosmétique filtrante photostable sous forme d'émulsion, destinée à protéger la peau du rayonnement UV, contenant en association, un filtre à large bande d'absorption photostable, de type particulier, dans la phase aqueuse et au moins un filtre UV-A photostable dans la phase huileuse. La présente invention est également relative à l'utilisation d'une telle composition pour la protection de la peau contre les rayons UV.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc aussi tout à fait souhaitable de filtrer le rayonnement UV-A.

Comme par ailleurs le rayonnement UV-A solaire est quantitativement plus important que le rayonnement UV-B solaire, on conçoit que l'on a intérêt à utiliser des compositions absorbant fortement dans la zone des UV-A, donc enrichies en composés absorbant dans cette région.

Le brevet français n° 2 528 420 décrit des composés absorbant fortement les rayons UV sur une large bande et notamment les dérivés du benzène [di(3-méthylidène camphre)] sulfonés en position 10 du camphre dont l'acide benzène 1,4-[di(3-méthylidène-10-camphosulfonique)], qu'on appelera ci-après composé 1. Ce composé photostable présente un maximum d'absorption dans l'UV-A à 342 nm et son absorption dans le domaine des UV-B correspondant aux rayons érythémateux est notable. Il constitue donc un filtre à large bande.

Ses groupements sulfoniques lui confèrent un caractère de solubilité dans l'eau. Malheureusement, pour des raisons d'origine cosmétique, il est souhaitable de ne pas utiliser ce composé à de très fortes concentrations.

Par ailleurs, ce composé ne possède pas une très bonne résistance à l'eau de mer et de piscine ni à la transpiration, ce qui peut nécessiter des applications répétées lors de l'exposition au soleil.

La demande GB 2 200 552 (L'OREAL) décrit une émulsion huile-dans-eau comprenant, dans la phase aqueuse, un filtre hydrosoluble à large bande qui est l'acide benzène 1,4-[di(3-méthylidène-10-camphosulfonique)] partiellement ou totalement neutralisé (composé 1) et, dans la phase huileuse, un filtre liposoluble UV-B dérivé du benzylidène camphre, à savoir le N-(2-éthylhexyl) 4-(3'-méthylidène camphre) benzène sulfonamide (composé 7).

La demanderesse a découvert qu'en associant ce filtre photostable à large bande dissous dans la phase aqueuse à un système filtrant photostable comprenant au moins un filtre UV-A dissous dans la phase huileuse d'une émulsion, on obtenait une très bonne protection contre le rayonnement solaire avec de plus une résistance à l'eau améliorée.

Le système filtrant UV-A photostable dissous dans la phase huileuse comprend au moins un des filtres UV-A suivants :
– le N-(2-éthylhexyl) 3-[(3'-méthoxy-4'-n-butoxy)benzylidène] 10-camphosulfonamide décrit dans le brevet français n° 2 529 887 (composé 2)
– le 4-(1,1-diméthyléthyl)-4'-méthoxydibenzoylméthane vendu sous la dénomination "PARSOL 1789" par la société GIVAUDAN (composé 3),
– le 4-isopropyldibenzoylméthane vendu sous la dénomination "EUSOLEX 8020" par la société MERCK (composé 4),
les deux composés 3 et 4 étant stabilisés photochimiquement par association avec un ou plusieurs des filtres liposolubles de $\lambda_{max}$ inférieur à 330 nm suivants :
– composé 5 : benzylidène camphre,
– composé 6 : p-méthylbenzylidène camphre vendu sous la dénomination "EUSOLEX 6300" par la société MERCK,
– composé 7 : N-(2-éthylhexyl) 4-(3'-méthylidène camphre) benzène sulfonamide décrit dans le brevet français n° 2 529 887,
– composé 8 : 3-méthoxy-4-n-butoxybenzylidène camphre décrit dans le brevet français n° 2 430 938.

La présente invention a donc pour objet une composition cosmétique filtrante photostable sous forme d'émulsion destinée à protéger la peau du rayonnement UV de longueurs d'onde comprises entre 280 et 380 nm, contenant en association, dans la phase aqueuse, un filtre photostable à large bande d'absorption constitué par l'acide benzène 1,4-[di(3-méthylidène-10-camphosulfonique)], partiellement ou totalement neutralisé, et dans la phase huileuse, un système photostable filtrant les UV-A comprenant au moins un des composés suivants : le N-(2-éthylhexyl) 3-[(3'-méthoxy-4'-n-butoxy)-benzylidène] 10-camphosulfonamide (composé 2), le 4-(1,1-diméthyléthyl)-4'-méthoxydibenzoylméthane (composé 3) et le 4-isopropyl dibenzoylméthane (composé 4), les deux derniers composés 3 et 4 étant stabilisés photochimiquement par un ou plusieurs filtres liposolubles de $\lambda_{max}$ inférieur à 330 nm choisis parmi le benzylidène camphre (composé 5), le p-méthylbenzylidène camphre (composé 6), le N-(2-éthylhexyl) 4-(3'-méthylidène camphre) benzène sulfonamide (composé 7) et le 3-méthoxy-4-n-butoxybenzylidène camphre (composé 8).

Un mode de réalisation de l'invention consiste à utiliser dans la phase huileuse, le N-(2-éthylhexyl) 3-[(3'-méthoxy-4'-n-butoxy)-benzylidène] 10-camphosulfonamide (composé 2) ou le 4-(1,1-diméthyléthyl)-4'-méthoxydibenzoylméthane (composé 3) stabilisé photochimiquement par le benzylidène camphre (composé 5), le N-(2-éthylhexyl)4-(3'-méthylidène camphre) benzène sulfonamide (composé 7) ou le 3-méthoxy-4-n-butoxybenzylidène camphre (composé 8), pris seuls ou en combinaison, ou encore une association des composés 2 et 3 avec un ou plusieurs des composés 5, 7 et 8.

L'émulsion photostable particulièrement préférée contient dans la phase huileuse le 4-(1,1-diméthyléthyl)-4'-méthoxydibenzoylméthane (composé 3) stabilisé photochimiquement par du p-méthylbenzylidène camphre (composé 6).

La présente invention a également pour objet un procédé de protection de l'épiderme humain contre les rayons UV de longueurs d'onde comprises entre 280 et 380 nm, consistant à appliquer sur la peau une quantité efficace de la composition cosmétique filtrante photostable précitée sous forme d'émulsion.

Pour neutraliser l'acide benzène 1,4-[di(3-méthylidène-10-camphosulfonique)], on peut utiliser les hydroxydes alcalins et plus particulièrement de sodium ou de potassium, l'ammoniaque, les alcanolamines, la triéthanolamine étant préférée.

Dans la composition cosmétique filtrante de l'invention, l'acide benzène 1,4-[di(3-méthylidène-10-camphosulfonique)] est présent dans des proportions comprises entre 0,1 et 10% en poids, et de préférence entre 0,5 et 5% en poids par rapport au poids total de la composition, avant neutralisation partielle ou totale.

Le système filtrant UV-A photostable ou stabilisé photochimiquement de la phase huileuse représente entre 0,1 et 15% en poids, et de préférence entre 0,5 et 10% en poids du poids total de la composition.

Dans le cas où l'on utilise le composé 3 ou le composé 4 stabilisés photochimiquement, on veillera à respecter un rapport en poids du ou des dérivé(s) de benzylidène camphre stabilisateur(s) (composés 5 à 8) au(x) dérivé(s) de dibenzoylméthane au moins égal à 1.

Pour des raisons de solubilisation des filtres dans la composition, ce rapport est de préférence inférieur ou égal à 6.

La composition cosmétique filtrante selon l'invention peut contenir d'autres filtres, et notamment le salicylate d'homomenthyle, qui est un filtre UV-B oléosoluble, dans des proportions pouvant aller jusqu'à 6% en poids par rapport au poids total de la composition.

La composition cosmétique filtrante selon l'invention peut se présenter sous forme de crème ou de lait et peut être conditionnée en aérosol. Elle a un pH compris entre 4 et 9 et de préférence entre 5,5 et 8. Il peut être ajusté à l'aide d'un agent alcalinisant ou acidifiant usuel.

Comme solvant de solubilisation des filtres liposolubles, on peut utiliser des corps gras tels que les huiles ou les cires minérales, animales ou végétales, les acides gras, les esters d'acides gras tels que les triglycérides d'acides gras ayant de 6 à 12 atomes de carbone, les alcools gras, les monoalcools ou les polyols inférieurs contenant de 2 à 6 atomes de carbone ou leurs mélanges. Les monoalcools ou polyols particulièrement préférés sont l'éthanol, l'isopropanol, le propylèneglycol et la glycérine.

La composition cosmétique filtrante selon l'invention destinée à protéger l'épiderme humain contre les rayons ultraviolets peut contenir les adjuvants cosmétiques usuels dans ce type de composition tels que des épaississants, des adoucissants, des humectants, des émulsionnants, des conservateurs, des anti-mousses, de la lanoline, des parfums, des propulseurs, des colorants et/ou des pigments ayant pour fonction de colorer la composition elle-même ou la peau, ou tout autre ingrédient habituellement utilisé en cosmétique.

L'invention a également pour objet une composition cosmétique filtrante contenant une concentration élevée en filtres définis ci-dessus, cette concentration pouvant aller jusqu'à 50%, les filtres étant dissous dans un savon. Cette composition, ou concentré, est diluable à l'eau ou dans une émulsion; on obtient ainsi une composition telle que décrite ci-dessus, destinée à être appliquée sur la peau.

Ce concentré contient de 10 à 17% de filtre hydrosoluble (composé 1) et de 15 à 33% de filtres oléosolubles (composés 2 à 8), dans une base constituée par un savon et de l'eau.

On utilisé de préférence un concentré contenant :
– jusqu'à 17% de composé 1

– jusqu'à 5,5 % de composé 3

– jusqu'à 27% de composé 6

– 12 à 15% d'un savon d'acide gras, le reste étant constitué par de l'eau.

Le savon est généralement du stéarate de triéthanolamine.

Les exemples suivants illustreront l'invention sans toutefois présenter un caractère limitatif.

## EXEMPLE 1

On prépare l'émulsion antisolaire suivante :

| | | |
|---|---|---|
| – Composé 1 | 5 | g |
| – Composé 3 | 2 | g |
| – Composé 7 | 4 | g |
| – Composé 5 | 2 | g |
| – Mélange d'alcool cétylstéarylique et d'alcool cétylstéarylique oxyéthyléné à 33 moles d'OE ("SINNOWAX AO" vendu par la société HENKEL) | 7,5 | g |
| – Mélange de mono- et de distéarate de glycérol ("GELEOL copeaux" vendu par la société GATTEFOSSE) | 2 | g |
| – Alcool cétylique | 1,8 | g |
| – Benzoate d'alcools gras en $C_{12}$-$C_{15}$ ("FINSOLV TN" vendu par la société FINETEX) | 15 | g |
| – Propylène glycol | 5 | g |
| – Glycérine | 8 | g |
| – Triéthanolamine | 3,4 | g |
| – Eau qsp | 100 | g |

On dissout les filtres liposolubles 3, 7 et 5 dans la phase grasse que l'on chauffe vers 75-80°C. La phase aqueuse contenant la glycérine, l'émulsionnant et le filtre hydrosoluble 1 est chauffée vers 75-80°C; sous vive agitation, on ajoute la phase grasse à la phase aqueuse. Après 15 minutes d'agitation vive, on laisse refroidir sous agitation modérée.

## EXEMPLE 2

On prépare la composition antisolaire suivante sous forme d'émulsion :

- Composé 1                                                                5     g
- Composé 2                                                                5     g
- Mélange d'alcool cétylstéarylique et d'alcool cétylstéarylique oxyéthyléné à 33 moles d'OE ("SINNOWAX AO" vendu par la société HENKEL)        7,5   g
- Mélange de mono et de distéarate de glycérol ("GELEOL copeaux" vendu par la société GATTEFOSSE)     2     g
- Alcool cétylique                                                         1,8   g
- Benzoate d'alcools gras en $C_{12}$-$C_{15}$ ("FINSOLV TN" vendu par la société FINETEX)              15    g
- Propylène glycol                                                         5     g
- Glycérine                                                                8     g
- Triéthanolamine                                                          3,4   g
- Eau                                                     qsp              100   g

Cette émulsion est préparée de la même façon que dans l'exemple 1.

## EXEMPLE 3

On prépare la composition antisolaire suivante sous forme d'émulsion :

- Composé 1                                                              4    g
- Composé 3                                                              1,5  g
- Composé 8                                                              5    g
- Mélange d'alcool cétyl stéarylique et d'alcool
  cétylstéarylique oxyéthyléné à 33 moles d'OE
  ("SINNOWAX AO" vendu par la société HENKEL)                           7,5  g
- Mélange de mono et de distéarate de glycérol
  ("GELEOL copeaux" vendu par la société GATTEFOSSE)   2    g
- Alcool cétylique                                                      1,8  g
- Benzoate d'alcools gras en $C_{12}-C_{15}$ ("FINSOLV TN"
  vendu par la société FINETEX)                                         15   g
- Propylène glycol                                                      5    g
- Glycérine                                                             8    g
- Triéthanolamine                                                       3,4  g
- Eau                                          qsp                      100  g

Cette émulsion est préparée de la même façon que dans l'exemple 1.

EXEMPLE 4

On prépare la composition filtrante suivante :

| | | |
|---|---|---|
| - Composé 1 | 3 | g |
| - Composé 3 | 3 | g |
| - Composé 6 | 3 | g |
| - Mélange d'alcool cétylstéarylique et d'alcool cétylstéarylique oxyéthyléné à 33 moles d'OE ("SINNOWAX AO" vendu par la société HENKEL) | 7 | g |
| - Mélange de mono et distéarate de glycérol non autoémulsionnable | 2 | g |
| - Benzoate d'alcools $C_{12}$-$C_{15}$ ("FINSOLV TN" vendu par la Société FINETEX) | 20 | g |
| - Alcool cétylique | 1,5 | g |
| - Huile de vaseline | 1,5 | g |
| - Polydiméthylsiloxane | 1,5 | g |
| - Glycérine | 20 | g |
| - Triéthanolamine | 1,8 | g |
| - Conservateurs | qs | |
| - Eau | qsp | 100 | g |

Cette émulsion est préparée de la même façon que dans l'exemple 1.

7

## EXEMPLE 5

On prépare l'émulsion antisolaire suivante :

| | | |
|---|---|---|
| – Composé 1 | 3 | g |
| – Composé 3 | 1,5 | g |
| – Composé 6 | 4,5 | g |
| – Mélange d'alcool cétylstéarylique et d'alcool cétylstéarylique oxyéthyléné à 33 moles d'OE ("SINNOWAX AO" vendu par la société HENKEL) | 7 | g |
| – Mélange de mono et distéarate de glycérol non autoémulsionnable | 2 | g |
| – Benzoate d'alcools $C_{12}$-$C_{15}$ ("FINSOLV TN" vendu par la Société FINETEX) | 20 | g |
| – Alcool cétylique | 1,5 | g |
| – Polydiméthylsiloxane | 1,5 | g |
| – Glycérine | 20 | g |
| – Triéthanolamine | 1,8 | g |
| – Conservateurs | qs | |
| – Eau | qsp 100 | g |

Cette émulsion est préparée de la même façon que dans l'exemple 1.

## EXEMPLE 6

On prépare l'émulsion antisolaire suivante :

| | |
|---|---|
| – Composé 1 | 3 g |
| – Parsol 1789 – composé 3 | 1 g |
| – Composé 6 | 5 g |
| – Salicylate d'homomenthyle | 4 g |
| – Mélange d'alcool cétylstéarylique et d'alcool cétylstéarylique oxyéthyléné à 33 moles d'OE ("SINNOWAX AO" vendu par la société HENKEL) | 7 g |
| – Mélange de mono et distéarate de glycérol non autoémulsionnable | 2 g |
| – Triglycérides d'acides caprique et caprylique ("MIGLYOL 812" vendu par la Société DYNAMIT NOBEL) | 30 g |
| – Polydiméthylsiloxane | 1,5 g |
| – Alcool cétylique | 1,5 g |
| – Conservateurs | qs |
| – Eau | qsp 100 g |

Cette émulsion est préparée de la même façon que dans l'exemple 1.

## EXEMPLE 7

On prépare le concentré suivant :

| | |
|---|---|
| – Composé 1 | 16,1 g |
| – Composé 3 | 5,4 g |
| – Composé 6 | 26,9 g |
| – Acide stéarique | 13,4 g |
| – Triéthanolamine | 10,7 g |
| – Eau | qsp 100 g |

Ce concentré se présente sous forme d'une pâte diluable en émulsion ou dans de l'eau.

## Revendications

**Revendications pour les Etats contractants suivants : AT, BE, CH, DE, FR, GB, GR, IT, LI, NL, SE**

1. Composition cosmétique filtrante photostable sous forme d'émulsion destinée à protéger la peau du rayonnement UV de longueurs d'onde comprises entre 280 et 380 nm, caractérisée par le fait qu'elle contient en association, dans la phase aqueuse, un filtre photostable à large bande d'absorption constitué par l'acide benzène 1,4-[di(3-méthylidène-10-camphosulfonique)] partiellement ou totalement neutralisé, et dans la phase huileuse, un système photostable filtrant les UV-A comprenant au moins l'un des composés suivants : le N-(2-éthylhexyl)3-[(3′-méthoxy-4′-n-butoxy)benzylidène] 10-camphosulfonamide (composé 2), le 4-(1,1-diméthyléthyl)-4′-méthoxydibenzoylméthane (composé 3) et le 4-isopropyl dibenzoylméthane (composé 4), ces deux derniers composés 3 et 4 étant stabilisés photochimiquement par au moins un filtre liposoluble de $\lambda_{max}$ inférieur à 330 nm choisi parmi les dérivés de benzylidène camphre suivants : benzylidène camphre (composé 5), p-méthylbenzylidène camphre (composé 6), N-(2-éthylhexyl) 4-(3′-méthylidène camphre) benzène sulfonamide (composé 7) et 3-méthoxy-4-n-butoxybenzylidène camphre (composé 8).

2. Composition cosmétique filtrante photostable selon la revendication 1, caractérisée par le fait qu'elle contient dans la phase huileuse, le N-(2-éthylhexyl) 3-[(3′-méthoxy-4′-n-butoxy)-benzylidène] 10-camphosulfonamide (composé 2) ou le 4-(1,1-diméthyléthyl)-4′-méthoxydibenzoylméthane (composé 3) stabilisé photochimiquement par au moins un composé choisi parmi le benzylidène camphre (composé 5), le N-(2-éthylhexyl)4-(3′-méthylidène camphre) benzène sulfonamide (composé 7) et le 3-méthoxy-4-n-butoxybenzylidène camphre (composé 8) ou une association des composés 2 et 3 avec un ou plusieurs des composés 5, 7 et 8.

3. Composition cosmétique filtrante photostable selon la revendication 1, caractérisée par le fait qu'elle contient dans la phase huileuse, le 4-(1,1-diméthyléthyl)-4′-méthoxydibenzoylméthane (composé 3) stabilisé photochimiquement par le p-méthyl benzylidène camphre (composé 6).

4. Composition cosmétique filtrante photostable selon la revendication 1, caractérisée par le fait qu'elle contient le 4-(1,1-diméthyléthyl)-4′-méthoxydibenzoylméthane ou le 4-isopropyl-dibenzoylméthane stabilisés photochimiquement par un ou plusieurs dérivés de benzylidène camphre choisis parmi les composés 5 à 8, le rapport en poids du ou des dérivé(s) de benzylidène camphre stabilisateurs au(x) dérivé(s) de dibenzoylméthane étant au moins égal à 1.

5. Composition cosmétique filtrante photostable selon la revendication 4, caractérisée par le fait que le rapport en poids du ou des dérivé(s) de benzylidène camphre stabilisateur(s) au(x) dérivé(s) de dibenzolyméthane est inférieur ou égal à 6.

6. Composition cosmétique filtrante phostostable selon l'une quelconque des revendications 1 à 5, caractérisée par le fait qu'elle contient en outre du salicylate d'homomenthyle dans des proportions allant jusqu'à 6% en poids par rapport au poids total de la composition.

7. Composition cosmétique filtrante photostable selon la revendication 1, caractérisée par le fait que l'acide benzène 1,4-[di-(3-méthylidène-10-camphosulfonique)] est partiellement ou totalement neutralisé par un hydroxyde alcalin, l'ammoniaque ou une alcanolamine.

8. Composition cosmétique filtrante photostable selon l'une quelconque des revendications 1 à 7, caractérisée par le fait qu'elle a un pH compris entre 4 et 9 et de préférence compris entre 5,5 et 8.

9. Composition cosmétique filtrante photostable selon l'une quelconque des revendications 1 à 8, caractérisée par le fait qu'elle contient 0,1 à 10% en poids, et de préférence 0,5 à 5% en poids par rapport au poids total de la composition, d'acide benzène 1,4-[di(3-méthylidène-10-campho sulfonique)] avant neutralisation partielle ou totale.

10. Composition cosmétique filtrante photostable selon l'une quelconque des revendications 1 à 9, caractérisée par le fait qu'elle contient 0,1 à 15% en poids, et de préférence 0,5 à 10% en poids par rapport au poids total de la composition, du système filtrant UV-A photostable ou photostabilisé chimiquement.

11. Composition cosmétique filtrante photostable selon l'une quelconque des revendications 1 à 10, caractérisée par le fait qu'elle se présente sous forme d'une crème ou d'un lait et peut être conditionnée en aérosol.

12. Composition cosmétique filtrante photostable selon l'une quelconque des revendications 1 à 11, caractérisée par le fait qu'elle contient un solvant de solubilisation des filtres liposolubles choisi parmi les huiles et cires minérales, animales ou végétales, les acides gras, les esters d'acides gras, les alcools gras et les monoalcools ou polyols inférieurs contenant 2 à 6 atomes de carbone.

13. Composition cosmétique filtrante photostable selon l'une quelconque des revendications 1 à 12, caractérisée par le fait qu'elle contient en outre des adjuvants cosmétiques choisis parmi les épaississants, les adoucissants, les humectants, les émulsionnants, les conservateurs, les anti-mousses, la lanoline, les parfums, les propulseurs, les colorants et les pigments.

14. Composition cosmétique filtrante photostable destinée à protéger la peau du rayonnement UV de longueurs d'onde comprises entre 280 et 380 nm, sous forme d'un concentré diluable à l'eau ou en émulsion, caractérisée par le fait qu'elle contient, dans une base constituée par un savon et de l'eau, un filtre photostable à large bande d'absorption constitué par l'acide benzène 1,4-[di(3-méthylidène-10-camphosulfonique)] partiellement ou totalement neutralisé et un système photostable filtrant les UV-A comprenant au moins l'un des composés suivants : le N-(2-éthylhexyl)3-[(3'-méthoxy-4'-n-butoxy)benzylidène] 10-camphosulfonamide (composé 2), le 4-(1,1-diméthyléthyl)-4'-méthoxydibenzoylméthane (composé 3) et le 4-isopropyl dibenzoylméthane (composé 4), ces deux derniers composés 3 et 4 étant stabilisés photochimiquement par au moins un filtre liposoluble de $\lambda_{max}$ inférieur à 330 nm choisi parmi les dérivés de benzylidène camphre suivants : benzylidène camphre (composé 5), p-méthylbenzylidène camphre (composé 6), N-(2-éthylhexyl) 4-(3'-méthylidène cemphre) benzène sulfonamide (composé 7) et 3-méthoxy-4-n-butoxybenzylidène camphre (composé 8), la concentration totale de filtres définis ci-dessus pouvant aller jusqu'à 50% en poids.

15. Composition cosmétique filtrante photostable sous forme de concentré selon la revendication 14 caractérisée par le fait qu'elle contient 10 à 17% en poids de filtre à large bande constitué par l'acide benzène 1,4-[di(3-méthylidène-10-camphosulfonique)] (composé 1), et 15 à 33% en poids de filtres constitués par les composés 2 à 8 selon la revendication 14.

16. Composition cosmétique filtrante photostable selon la revendication 15, caractérisée par le fait qu'elle contient 10 à 17% en poids de filtre constitué par le composé 1, jusqu'à 5,5% en poids de composé 3, jusqu'à 27% en poids de composé 6, 12 à 15% en poids d'un savon d'acide gras, le reste étant constitué par de l'eau.

**Revendications pour l'Etat contractant suivant : ES**

1. Composition cosmétique filtrante photostable sous forme d'émulsion destinée à protéger la peau du rayonnement UV de longueurs d'onde comprises entre 280 et 380 nm, caractérisée par le fait qu'elle contient en association, dans la phase aqueuse, un filtre photostable à large bande d'absorption constitué par l'acide benzène 1,4-[di(3-méthylidène-10-camphosulfonique)] partiellement ou totalement neutralisé, et dans la phase huileuse, un système photostable filtrant les UV-A comprenant au moins l'un des composés suivants : le N-(2-éthylhexyl)3-[(3'-méthoxy-4'-n-butoxy)benzylidène] 10-camphosulfonamide (composé 2), le 4-(1,1-diméthyléthyl)-4'-méthoxydibenzoylméthane (composé 3) et le 4-isopropyl dibenzoylméthane (composé 4), ces deux derniers composés 3 et 4 étant stabilisés photochimiquement par au moins un filtre liposoluble de $\lambda_{max}$ inférieur à 330 nm choisi parmi les dérivés de benzylidène camphre suivants : benzylidène camphre (composé 5), p-méthylbenzylidène camphre (composé 6), N-(2-éthylhexyl) 4-(3'-méthylidène camphre) benzène sulfonamide (composé 7) et 3-méthoxy-4-n-butoxybenzylidène camphre (composé 8).

2. Composition cosmétique filtrante photostable selon la revendication 1, caractérisée par le fait qu'elle contient dans la phase huileuse, le N-(2-éthylhexyl) 3-[(3'-méthoxy-4'-n-butoxy)-benzylidène] 10-camphosulfonamide (composé 2) ou le 4-(1,1-diméthyléthyl)-4'-méthoxydibenzoylméthane (composé 3) stabilisé photochimiquement par au moins un composé choisi parmi le benzylidène camphre (composé 5), le N-(2-éthylhexyl)4-(3'-méthylidène camphre) benzène sulfonamide (composé 7) et le 3-méthoxy-4-n-butoxybenzylidène camphre (composé 8) ou une association des composés 2 et 3 avec un ou plusieurs des composés 5, 7 et 8.

3. Composition cosmétique filtrante photostable selon la revendication 1, caractérisée par le fait qu'elle contient dans la phase huileuse, le 4-(1,1-dimithyléthyl)-4'-méthoxydibenzoylméthane (composé 3) stabilisé photochimiquement par le p-méthyl benzylidène camphre (composé 6).

4. Composition cosmétique filtrante photostable selon la revendication 1, caractérisée par le fait qu'elle contient le 4-(1,1-diméthyléthyl)-4'-méthoxydibenzoylméthane ou le 4-isopropyldibenzoylméthane stabilisés, photochimiquement par un ou plusieurs dérivés de benzylidène camphre choisis parmi les composés 5 à 8, le rapport en poids du ou des dérivé(s) de benzylidène camphre stabilisateurs au(x) dérivé(s) de dibenzoylméthane étant au moins égal à 1.

5. Composition cosmétique filtrante photostable selon la revendication 4, caractérisée par le fait que le rapport en poids du ou des dérivé(s) de benzylidène camphre stabilisateur(s) au(x) dérivé(s) de dibenzolyméthane est inférieur ou égal à 6.

6. Composition cosmétique filtrante phostostable selon l'une quelconque des revendications 1 à 5, caractérisée par le fait qu'elle contient en outre du salicylate d'homomenthyle dans des proportions allant jusqu'à 6% en poids par rapport au poids total de la composition.

7. Composition cosmétique filtrante photostable selon la revendication 1, caractérisée par le fait que l'acide benzène 1,4-[di(3-méthylidène-10-camphosulfonique)] est partiellement ou totalement neutralisé par un hydroxyde alcalin, l'ammoniaque ou une alcanolamine.

8. Composition cosmétique filtrante photostable selon l'une quelconque des revendications 1 à 7, caractérisée par le fait qu'elle a un pH compris entre 4 et 9 et de préférence compris entre 5,5 et 8.

9. Composition cosmétique filtrante photostable selon l'une quelconque des revendications 1 à 8, caractérisée par le fait qu'elle contient 0,1 à 10% en poids, et de préférence 0,5 à 5% en poids par rapport au poids total de la composition, d'acide benzène 1,4-[di(3-méthylidène-10-campho sulfonique)] avant neutralisation partielle ou totale.

10. Composition cosmétique filtrante photostable selon l'une quelconque des revendications 1 à 9, caractérisée par le fait qu'elle contient 0,1 à 15% en poids, et de préférence 0,5 à 10% en poids par rapport au poids total de la composition, du système filtrant UV-A photostable ou photostabilisé chimiquement.

11. Composition cosmétique filtrante photostable selon l'une quelconque des revendications 1 à 10, caractérisée par le fait qu'elle se présente sous forme d'une crème ou d'un lait et peut être conditionnée en aérosol.

12. Composition cosmétique filtrante photostable selon l'une quelconque des revendications 1 à 11, caractérisée par le fait qu'elle contient un solvant de solubilisation des filtres liposolubles choisi parmi les huiles et cires minérales, animales ou végétales, les acides gras, les esters d'acides gras, les alcools gras et les monoalcools ou polyols inférieurs contenant 2 à 6 atomes de carbone.

13. Composition cosmétique filtrante photostable selon l'une quelconque des revendications 1 à 12, caractérisée par le fait qu'elle contient en outre des adjuvants cosmétiques choisis parmi les épaississants, les adoucissants, les humectants, les émulsionnants, les conservateurs, les anti-mousses, la lanoline, les parfums, les propulseurs, les colorants et les pigments.

14. Composition cosmétique filtrante photostable destinée à protéger la peau du rayonnement UV de longueurs d'onde comprises entre 280 et 380 nm, sous forme d'un concentré diluable à l'eau ou en émulsion, caractérisée par le fait qu'elle contient, dans une base constituée par un savon et de l'eau, un filtre photostable à large bande d'absorption constitué par l'acide benzène 1,4-[di(3-méthylidène-10-camphosulfonique)] partiellement ou totalement neutralisé et un système photostable filtrant les UV-A comprenant au moins l'un des composés suivants : le N-(2-éthylhexyl)3-[(3'-méthoxy-4'-n-butoxy)benzylidène] 10-camphosulfonamide (composé 2), le 4-(1,1-diméthyléthyl)-4'-méthoxydibenzoylméthane (composé 3) et le 4-isopropyl dibenzoylméthane (composé 4), ces deux derniers composés 3 et 4 étant stabilisés photochimiquement par au moins un filtre liposoluble de $\lambda_{max}$ inférieur à 330 nm choisi parmi les dérivés de benzylidène camphre suivants : benzylidène camphre (composé 5), p-méthylbenzylidène camohre (composé 6), N-(2-éthylhexyl) 4-(3'-méthylidène camphre) benzène sulfonamide (composé 7) et 3-méthoxy-4-n-butoxybenzylidène camphre (composé 8), la concentration totale de filtres définis ci-dessus pouvant aller jusqu'à 50% en poids.

15. Composition cosmétique filtrante photostable sous forme de concentré selon la revendication 14, caractérisée par le fait qu'elle contient 10 à 17% en poids de filtre à large bande constitué par l'acide benzène 1,4-[di(3-méthylidène-10-camphosulfonique)] (composé 1), et 15 à 33% en poids de filtres constitués par les composés 2 à 8 selon la revendication 14.

16. Composition cosmétique filtrante photostable selon la revendication 15, caractérisée par le fait qu'elle contient 10 à 17% en poids de filtre constitué par le composé 1, jusqu'à 5,5% en poids de composé 3, jusqu'à 27% en poids de composé 6, 12 à 15% en poids d'un savon d'acide gras, le reste étant constitué par de l'eau.

17. Procédé de préparation d'une composition cosmétique filtrante photostable sous forme d'émulsion destinée à protéger la peau du rayonnement UV de longueurs d'onde comprises entre 280 et 380 nm, caractérisé par le fait qu'il consiste :

a) dans une première étape, à dissoudre dans une phase aqueuse, 0,1 à 10% en poids, d'un filtre photostable à large bande d'absorption constitué par l'acide benzène-1,4-[di(3-méthylidène-10-camphosulfonique)], qu'on neutralise partiellement ou totalement par un hydroxyde alcalin, l'ammoniaque ou une alcanolamine, et à ajouter ensuite l'émulsionnant ;

b) dans une seconde étape, à dissoudre dans une phase grasse comprenant des huiles ou cires minérales, animales ou végétales, des acides gras, des esters d'acides gras, des alcools gras, des monoalcools ou polyols inférieurs contenant 2 à 6 atomes de carbone ou leur mélange, 0,1 à 15% en poids d'un système photostable filtrant les UV-A comprenant au moins l'un des composés suivants : le N-(2-éthylhexyl)3-[(3'-méthoxy-4'-n-butoxy)benzylidène] 10-camphosulfonamide (composé 2), le (composé 2), le 4-(1,1-diméthyléthyl)-4'-méthoxydibenzoylméthane (composé 3) et le 4-isopropyl dibenzoylméthane (composé 4), ces deux derniers composés 3 et 4 étant stabilisés photochimiquement par au moins un filtre liposoluble le $\lambda_{max}$ inférieur à 330 nm choisi parmi les dérivés de benzylidène camphre suivants : benzylidène camphre (composé 5), p-méthylbenzylidène camphre (composé 6), N-(2-éthylhexyl) 4-(3'-méthylidène camphre) benzène sulfonamide (composé 7) et 3-méthoxy-4-n-butoxybenzylidène camphre (composé 8), les pourcentages de filtres utilisés dans les étapes a) et b) étant calculés sur la base du poids total de l'émulsion ;

c) dans une troisième étape, à chauffer indépendamment les phases aqueuse et grasse à une température d'environ 75-80°C, et sous vive agitation, à ajouter la phase grasse à la phase aqueuse.

18. Procédé de préparation d'une composition cosmétique filtrante photostable destinée à protéger la peau du rayonnement UV de longueurs d'onde comprises entre 280 et 380 nm, sous forme d'un concentré diluable à l'eau ou en émulsion, caractérisé par le fait qu'il consiste à dissoudre dans une base constituée par un savon et de l'eau, 10 à 17% en poids d'un filtre photostable à large bande d'absorption constitué par l'acide benzène-1,4-[di(3-méthylidène-10-camphosulfonique)] et 15 à 33% en poids d'un système photostable filtrant les UV-A comprenant au moins l'un des composés suivants : le N-(2-éthylhexyl)3-[(3'-méthoxy-4'-n-butoxy)benzyliène] 10-camphosulfonamide (composé 2), le 4-(1,1-diméthyléthyl)-4'-méthoxydibenzoylmétane (composé 3) et le 4-isopropyl dibenzoylméthane (composé 4), ces deux derniers composés 3 et 4 étant stabilisés photochimiquement par au moins un filtre liposoluble de $\lambda_{max}$ inférieur à 330 nm choisi parmi les dérivés de benzylidène camphre suivants : benzylidène camphre (composé 5), p-méthylbenzylidène camphre (composé 6), N-(2-éthylhexyl) 4-(3'-méthylidène camphre) benzène sulfonamide (composé 7) et 3-méthoxy-4-n-butoxybenzylidène camphre (composé 8), les pourcentages de filtres utilisés étant calculés sur la base du poids total du concentré.

## Patentansprüche

## Patentansprüche für folgende Vertragsstaaten : AT, BE, CH, DE, FR, GB, GR, IT, LI, NL, SE

1. Photostabile, lichtfiltrierende, kosmetische Zusammensetzung in Form einer Emulsion zum Schutz der Haut vor UV-Strahlung im Wellenlängenbereich von 280 bis 380 nm,

dadurch gekennzeichnet, daß sie

in Kombination in wäßriger Phase eine photostabile Filtersubstanz mit breiter Absorptionsbande, bestehend aus teilweise oder vollständig neutralisierter Benzol-1,4-[di-(3-methyliden-10 -camphosulfonsäure)], und in der Ölphase ein photostabiles UV-A-filtrierendes System enthält, welches mindestens eine der folgenden Verbindungen umfaßt: N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3′-methylidenkampfer)-benzolsulfonamid (Verbindung 7) und 3-Methoxy-4-n-butoxybenzylidenkampfer (Verbindung 8).

2. Photostabile, lichtfiltrierende, kosmetische Zusammensetzung nach Anspruch 1, dadurch gekennzeichnet, daß sie in der öligen Phase N-(2-Ethylhexyl)-3-[(3′-methoxy-4′-n-butoxy)-benzyliden]-10-camphosulfonamid (Verbindung 2) oder 4-(1,1-Dimethylethyl)-4′-methoxydibenzoylmethan (Verbindung 3) enthält, photochemisch stabilisiert durch wenigstens eine Verbindung, ausgewählt unter Benzylidenkampfer (Verbindung 5), N-(2-Ethylhexyl)-4-(3′-methylidenkampfer)-benzolsulfonamid (Verbindung 7) und 3-Methoxy-4-n-butoxybenzylidenkampfer (Verbindung 8), oder eine Kombination der Verbindungen 2 und 3 mit einer oder mehreine Kombination der Verbindungen 2 und 3 mit einer oder mehreren der Verbindungen 5, 7 und 8.

3. Photostabile, lichtfiltrierende, kosmetische Zusammensetzung nach Anspruch 1, dadurch gekennzeichnet, daß sie in der öligen Phase 4-(1,1-Dimethylethyl)-4′-methoxydibenzoylmethan (Verbindung 3), photochemisch stabilisiert durch p-Methylbenzylidenkampfer (Verbindung 6), enthält.

4. Photostabile, lichtfiltrierende, kosmetische Zusammensetzung nach Anspruch 1, dadurch gekennzeichnet, daß sie 4-(1,1-Dimethylethyl)-4′-methoxydibenzoylmethan oder 4-Isopropyldibenzoylmethan , photochemisch stabilisiert durch ein oder mehrere Benzylidenkampferderivate, ausgewählt unter den Verbindungen 5 bis 8 enthält, wobei das Gewichtsverhältnis des Derivats oder der Derivate der Benzylidenkampfer-Stabilisatoren zu dem (den) Dibenzoylmethanderivat(en) wenigstens gleich 1 ist.

5. Photostabile, lichtfiltrierende, kosmetische Zusammensetzung nach Anspruch 4, dadurch gekennzeichnet, daß das Gewichtsverhältnis des Derivats oder der Derivate der Benzylidenkampfer-Stabilisatoren zu dem (den) Dibenzoylmethanderivat(en) kleiner oder gleich 6 ist.

6. Photostabile, lichtfiltrierende, kosmetische Zusammensetzung nach einem der Ansprüche 1 bis 5, dadurch gekennzeichent, daß sie außerdem Homomenthylsalicylat in einem Anteil von bis zu 6 Gew.-%, bezogen auf das Gesamtgewicht der Zusammensetzung, enthält.

7. Photostabile, lichtfiltrierende, kosmetische Zusammensetzung nach Anspruch 1, dadurch gekennzeichnet, daß die Benzol-1,4-[di-(3-methyliden-10-camphosulfonsäure)] teilweise oder vollständig neutralisiert ist mit einem alkalischen Hydroxid, Ammoniak oder einem Alkanolamin.

8. Photostabile, lichtfiltrierende, kosmetische Zusammensetzung nach einem der Ansprüche 1 bis 7, dadurch gekennzeichnet, daß sie einen pH im Bereich von 4 bis 9 und vorzugsweise im Bereich von 5,5 bis 8 aufweist.

9. Photostabile, lichtfiltrierende, kosmetische Zusammensetzung nach einem der Ansprüche 1 bis 8, dadurch gekennzeichnet, daß sie 0,1 - 10 Gew.-% und vorzugsweise 0,5 - 5 Gew.-%, bezogen auf das Gesamtgewicht der Zusammensetzung, Benzol-1,4-[di-(3-methyliden-10-camphosulfonsäure)] vor partieller oder vollständiger Neutralisation enthält.

10. Photostabile, lichtfiltrierende, kosmetische Zusammensetzung nach einem der Ansprüche 1 bis 9, dadurch gekennzeichnet, daß sie 0,1 - 15 Gew.-% und vorzugsweise 0,5 - 10 Gew.-%, bezogen auf das Gesamtgewicht der Zusammensetzung, ein photostabiles oder chemisch photostabilisiertes UV-A-Filtersystem enthält.

11. Photostabile, lichtfiltrierende, kosmetische Zusammensetzung nach einem der Ansprüche 1 bis 10, dadurch gekennzeichnet, daß sie in Form einer Creme oder Milch vorliegt oder als Aerosol konditioniert ist.

12. Photostabile, lichtfiltrierende, kosmetische Zusammensetzung nach einem der Ansprüche 1 bis 11, dadurch gekennzeichnet, daß sie ein Lösungsmittel zur Solubilisierung fettlöslicher Filter enthält, ausgewählt unter mineralischen, tierischen oder pflanzlichen Ölen oder Wachsen, Fettsäuren, Fettsäureestern, Fettalkoholen und niedrigen Monoalkoholen oder Polyolen mit 2 bis 6 Kohlenstoffatomen.

13. Photostabile, lichtfiltrierende, kosmetische Zusammensetzung nach einem der Ansprüche 1 bis 12, dadurch gekennzeichnet, daß sie außerdem kosmetische Adjuvantien enthält, die ausgewählt sind unter Verdickungsmitteln, weichmachenden Mitteln, Feuchthaltemitteln, emulgierenden Mitteln, konservierenden Mitteln, Antischaummitteln, Lanolin, Parfüms, Treibmitteln, Farbstoffen und Pigmenten.

14. Photostabile, lichtfiltrierende, kosmetische Zusammensetzung zum Schutz der Haut vor UV-Strahlung im Wellenlängenbereich von 280 bis 380 nm in Form eines mit Wasser verdünnbaren Konzentrats oder einer Emulsion, dadurch gekennzeichnet, daß sie in einem Träger, bestehend aus einer Seife und Wasser eine photostabile Filtersubstanz mit breiter Absorptionsbande enthält, bestehend aus teilweise oder vollständig neutralisierter Benzol-1,4-[di-(3-methyliden-10-camphosulfonsäure)] und einem photostabilen UV-A-Filtersystem, umfassend wenigstens eine der folgenden Verbindungen: N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3'-methylidenkampfer)-benzolsulfonamid (Verbindung 7) und 3-Methoxy-4-n-butoxybenzylidenkampfer (Verbindung 8), wobei die Gesamtkonzentration der oben definierten Filter bis zu 50 Gew.-% betragen kann.

15. Photostabile, lichtfiltrierende, kosmetische Zusammensetzung in Form eines Konzentrats nach Anspruch 14, dadurch gekennzeichnet, daß sie 10 bis 17 Gew.-% der Breitband-Filtersubstanz enthält, bestehend aus Benzol-1,4-[di-(3-methyliden-10-campholsulfonsäure)] (Verbindung 1) und 15 bis 33 Gew.-% Filtersubstanzen, bestehend aus den Verbindungen 2 bis 8 gemäß Anspruch 14.

16. Photostabile, lichtfiltrierende, kosmetische Zusammensetzung nach Anspruch 15, dadurch gekennzeichnet, daß sie 10 bis 17 Gew.-% einer Filtersubstanz, bestehend aus Verbindung 1, bis zu 5,5 Gew.-% der Verbindung 3, bis zu 27 Gew.-% der Verbindung 6 und 12 bis 15 Gew.-% einer verseiften Fettsäure enthält, und wobei der Rest Wasser ist.

## Patentansprüche für folgenden Vertragsstaat : ES

1. Photostabile, lichtfiltrierende, kosmetische Zusammensetzung in Form einer Emulsion zum Schutz der Haut vor UV-Strahlung im Wellenlängenbereich von 280 bis 380 nm, dadurch gekennzeichnet, daß sie in Kombination in wäßriger Phase eine photostabile Filtersubstanz mit breiter Absorptionsbande, bestehend aus teilweise oder vollständig neutralisierter Benzol-1,4-[di-(3-methyliden-10 -camphosulfonsäure)], und in der Ölphase ein photostabiles UV-A-filtrierendes System enthält, welches mindestens eine der folgenden Verbindungen umfaßt: N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3'-methylidenkampfer)-benzolsulfonamid (Verbindung 7) und 3-Methoxy-4-n-butoxybenzylidenkampfer (Verbindung 8).

2. Photostabile, lichtfiltrierende, kosmetische Zusammensetzung nach Anspruch 1, dadurch gekennzeichnet, daß sie in der öligen Phase N-(2-Ethylhexyl)-3-[(3'-methoxy-4'-n-butoxy)-benzyliden]-10-camphosulfonamid (Verbindung 2) oder 4-(1,1-Dimethylethyl)-4'-methoxydibenzoylmethan (Verbindung 3) enthält, photochemisch stabilisiert durch wenigstens eine Verbindung, ausgewählt unter Benzylidenkampfer (Verbindung 5), N-(2-Ethylhexyl)-4-(3'-methylidenkampfer)-benzolsulfonamid (Verbindung 7) und 3-Methoxy-4-n-butoxybenzylidenkampfer (Verbindung 8), oder eine Kombination der Verbindungen 2 und 3 mit einer oder mehreren der Verbindungen 5, 7 und 8.

3. Photostabile, lichtfiltrierende, kosmetische Zusammensetzung nach Anspruch 1, dadurch gekennzeichnet, daß sie in der öligen Phase 4-(1,1-Dimethylethyl)-4'-methoxydibenzoylmethan (Verbindung 3), photochemisch stabilisiert durch p-Methylbenzylidenkampfer (Verbindung 6), enthält.

4. Photostabile, lichtfiltrierende, kosmetische Zusammensetzung nach Anspruch 1, dadurch gekennzeichnet, daß sie 4-(1,1-Dimethylethyl)-4'-methoxydibenzoylmethan oder 4-Isopropyldibenzoylmethan, photochemisch stabilisiert durch ein oder mehrere Benzylidenkampferderivate, ausgewählt unter den Verbindungen 5 bis 8 enthält, wobei das Gewichtsverhältnis des Derivats oder der Derivate der Benzylidenkampfer-Stabilisatoren zu dem (den) Dibenzoylmethanderivat(en) wenigstens gleich 1 ist.

5. Photostabile, lichtfiltrierende, kosmetische Zusammensetzung nach Anspruch 4, dadurch gekennzeichnet, daß das Gewichtsverhältnis des Derivats oder der Derivate der Benzylidenkampfer-Stabilisatoren zu dem (den) Dibenzoylmethanderivat(en) kleiner oder gleich 6 ist.

6. Photostabile, lichtfiltrierende, kosmetische Zusammensetzung nach einem der Ansprüche 1 bis 5, dadurch gekennzeichent, daß sie außerdem Homomenthylsalicylat in einem Anteil von bis zu 6 Gew.-%, bezogen auf das Gesamtgewicht der Zusammensetzung, enthält.

7. Photostabile, lichtfiltrierende, kosmetische Zusammensetzung nach Anspruch 1, dadurch gekennzeichnet, daß die Benzol-1,4-[di-(3-methyliden-10-camphosulfonsäure)] teilweise oder vollständig neutralisiert ist mit einem alkalischen Hydroxid, Ammoniak oder einem Alkanolamin.

8. Photostabile, lichtfiltrierende, kosmetische Zusammensetzung nach einem der Ansprüche 1 bis 7, dadurch gekennzeichnet, daß sie einen pH im Bereich von 4 bis 9 und vorzugsweise im Bereich von 5,5 bis 8 aufweist.

9. Photostabile, lichtfiltrierende, kosmetische Zusammensetzung nach einem der Ansprüche 1 bis 8, dadurch gekennzeichnet, daß sie 0,1 - 10 Gew.-% und vorzugsweise 0,5 - 5 Gew.-%, bezogen auf das Gesamtgewicht der Zusammensetzung, Benzol-1,4-[di-(3-methyliden-10-camphosulfonsäure)] vor partieller oder vollständiger Neutralisation enthält.

10. Photostabile, lichtfiltrierende, kosmetische Zusammensetzung nach einem der Ansprüche 1 bis 9, dadurch gekennzeichnet, daß sie 0,1 - 15 Gew.-% und vorzugsweise 0,5 - 10 Gew.-%, bezogen auf das Gesamtgewicht der Zusammensetzung, ein photostabiles oder chemisch photostabilisiertes UV-A-Filtersystem enthält.

11. Photostabile, lichtfiltrierende, kosmetische Zusammensetzung nach einem der Ansprüche 1 bis 10, dadurch gekennzeichnet, daß sie in Form einer Creme oder Milch vorliegt oder als Aerosol konditioniert ist.

12. Photostabile, lichtfiltrierende, kosmetische Zusammensetzung nach einem der Ansprüche 1 bis 11, dadurch gekennzeichnet, daß sie ein Lösungsmittel zur Solubilisierung fettlöslicher Filter enthält, ausgewählt unter mineralischen, tierischen oder pflanzlichen Ölen oder Wachsen, Fettsäuren, Fettsäureestern, Fettalkoholen und niedrigen Monoalkoholen oder Polyolen mit 2 bis 6 Kohlenstoffatomen.

13. Photostabile, lichtfiltrierende, kosmetische Zusammensetzung nach einem der Ansprüche 1 bis 12, dadurch gekennzeichnet, daß sie außerdem kosmetische Adjuvantien enthält, die ausgewählt sind unter Verdickungsmitteln, weichmachenden Mitteln, Feuchthaltemitteln, emulgierenden Mitteln, konservierenden Mitteln, Antischaummitteln, Lanolin, Parfüms, Treibmitteln, Farbstoffen und Pigmenten.

14. Photostabile, lichtfiltrierende, kosmetische Zusammensetzung zum Schutz der Haut vor UV-Strahlung im Wellenlängenbereich von 280 bis 380 nm in Form eines mit Wasser verdünnbaren Konzentrats oder einer Emulsion, dadurch gekennzeichnet, daß sie in einem Träger, bestehend aus einer Seife und Wasser eine photostabile Filtersubstanz mit breiter Absorptionsbande enthält, bestehend aus teilweise oder vollständig neutralisierter Benzol-1,4-[di-(3-methyliden-10-camphosulfonsäure)] und einem photostabilen UV-A-Filtersystem, umfassend wenigstens eine der folgenden Verbindungen:
N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3'-methylidenkampfer)-benzolsulfonamid (Verbindung 7) und 3-Methoxy-4-n-butoxybenzylidenkampfer (Verbindung 8),
wobei die Gesamtkonzentration der oben definierten Filter bis zu 50 Gew.-% betragen kann.

15. Photostabile, lichtfiltrierende, kosmetische Zusammensetzung in Form eines Konzentrats nach Anspruch 14, dadurch gekennzeichnet, daß sie 10 bis 17 Gew.-% der Breitband-Filtersubstanz enthält, bestehend aus Benzol-1,4-[di-(3-methyliden-10-campholsulfonsäure)] (Verbindung 1) und 15 bis 33 Gew.-% Filtersubstanzen, bestehend aus den Verbindungen 2 bis 8 gemäß Anspruch 14.

16. Photostabile, lichtfiltrierende, kosmetische Zusammensetzung nach Anspruch 15, dadurch gekennzeichnet, daß sie 10 bis 17 Gew.-% einer Filtersubstanz, bestehend aus Verbindung 1, bis zu 5,5 Gew.-% der Verbindung 3, bis zu 27 Gew.-% der Verbindung 6 und 12 bis 15 Gew.-% einer verseiften Fettsäure enthält, und wobei der Rest Wasser ist.

17. Verfahren zur Herstellung einer photostabilen, lichtfiltrierenden, kosmetischen Zusammensetzung in Form einer Emulsion zum Schutz der Haut vor UV-Strahlung im Wellenlängenbereich von 280 - 380 nm, dadurch gekennzeichnet, daß man:
a) in einem ersten Schritt in einer wäßrigen Phase 0,1 - 10 Gew.-% einer photostabilen Filtersubstanz mit breiter Absorptionsbande, bestehend aus Benzol-1,4-[di-(3-methyliden-10-campholsulfonsäure)] löst, das man teilweise oder vollständig mit einem alkalischen Hydroxid, Ammoniak oder einem Alkanolamin neutralisiert und ein Emulgiermittel zugibt;
b) in einem zweiten Schritt in einer Fettphase, umfassend mineralische, tierische oder pflanzliche Öle oder Wachse, Fettsäuren, Fettsäureester, Fettalkohole, niedrige Monoalkohole oder Polyole mit 2 bis 6 Kohlenstoffatomen oder Gemische davon, 0,1 bis 15 Gew.-% eines photostabilen UV-A-filtrierenden Systems löst, umfassend wenigstens eine der folgenden Verbindungen: N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3'-methylidenkampfer)-benzolsulfonamid (Verbindung 7) und 3-Methoxy-4-n-butoxybenzylidenkampfer (Verbindung 8), wobei die Prozentangaben der verwendeten Filtersubstanzen in den Stufen a) und b) auf Grundlage des Gesamtgewichts der Emulsion berechnet ist;
c) in einem dritten Schritt die wäßrige und die Fettphase auf eine Temperatur von etwa 75 - 80°C unabhängig voneinander erwärmt und unter kräftigem Rühren die Fettphase zur wäßrigen Phase hinzugibt.

18. Verfahren zur Herstellung einer photostabilen, lichtfiltrierenden, kosmetischen Zusammensetzung zum Schutz der Haut vor UV-Strahlung im Wellenlängenbereich von 280 bis 380 nm in Form eines mit Wasser verdünnbaren Konzentrats oder einer Emulsion, dadurch gekennzeichnet, daß man in einem Träger, bestehend aus einer Seife und Wasser 10 bis 17 Gew.-% einer photostabilen Filtersubstanz mit breiter Absorptionsbande, bestehend aus Benzol-1,4-[di-(3-methyliden-10-camphosulfonsäure)] und 15 bis 33 Gew.-% eines photostabilen UV-A-Filtersystems löst, welches wenigstens eine der folgenden Verbindungen umfaßt: N-(2-Ethylhexyl)-3-[(3'-methoxy-4'-n-butoxy)-benzyliden]-10-camphosulfonamid (Verbindung 2), 4-(1,1-Dimethylethyl)-4'-methoxydibenzoylmethan (Verbindung 3) und 4-Isopropyldibenzoylmethan (Verbindung 4), wobei die beiden letzten Verbindungen 3 und 4 photochemisch stabilisiert sind durch mindestens eine fettlösliche Filtersubstanz mit $\lambda_{max}$ kleiner als 330 nm, ausgewählt unter folgenden Benzylidenkampferderivaten: Benzylidenkampfer (Verbindung 5), p-Methylbenzylidenkampfer (Verbindung 6), N-(2-Ethylhexyl)-4-(3'-methylidenkampfer)-benzolsulfonamid (Verbindung 7) und 3-Methoxy-4-n-butoxybenzylidenkampfer (Verbindung 8), wobei die Prozentangaben für die verwendeten Filtersubstanzen auf Basis des Gesamtgewichts des Konzentrats berechnet sind.

## Claims

### Claims for the following Contracting States : AT, BE, CH, DE, FR, GB, GR, IT, LI, NL, SE

1. Photostable screening cosmetic composition in the form of an emulsion intended for protecting the skin from UV radiation of wavelengths between 280 and 380 nm, characterised in that it contains in combination, in the aqueous phase, a photostable screening agent with a broad absorption band consisting of 1,4-benzene[di(3-methylidene-10-camphosulphonic)] acid which is partially or totally neutralised, and, in the oily phase, a photostable system screening out UV-A radiation, comprising at least one of the following compounds: N-(2-ethylhexyl)-3-[(3'-methoxy-4'-n-butoxy)benzylidene]-10-camphosulphonamide (compound 2), 4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8).

2. Photostable screening cosmetic composition according to Claim 1, characterised in that it contains, in the oily phase, N-(2-ethylhexyl)-3-[(3′-methoxy-4′-n-butoxy)benzylidene]-10-camphosulphonamide (compound 2) or 4-(1,1-dimethylethyl)-4′-methoxydibenzoylmethane (compound 3) which is photochemically stabilised by at least one compound chosen from benzylidenecamphor (compound 5), N-(2-ethylhexyl)-4-(3′-methylidenecamphor)benzenesulphonamide (compound 7) and 3-methoxy-4-n-butoxybenzylidenecamphor (compound 8) or a combination of the compounds 2 and 3 with one or more of the compounds 5, 7 and 8.

3. Photostable screening cosmetic composition according to Claim 1, characterised in that it contains, in the oily phase, 4-(1,1-dimethylethyl)-4′-methoxydibenzoylmethane (compound 3) which is photochemically stabilised by p-methylbenzylidenecamphor (compound 6).

4. Photostable screening cosmetic composition according to Claim 1, characterised in that it contains 4-(1,1-dimethylethyl)-4′-methoxydibenzoylmethane or 4-isopropyldibenzoylmethane, these compounds being photochemically stabilised by one or more benzylidenecamphor derivatives chosen from the compounds 5 to 8, the ratio by weight of the stabilising benzylidenecamphor derivative(s) to the dibenzoylmethane derivative(s) being not less than 1.

5. Photostable screening cosmetic composition according to Claim 4, characterised in that the ratio by weight of the stabilising benzylidenecamphor derivative(s) to the dibenzoylmethane derivative(s) is not more than 6.

6. Photostable screening cosmetic composition according to any one of Claims 1 to 5, characterised in that it in addition contains homomenthyl salicylate in proportions ranging up to 6% by weight relative to the total weight of the composition.

7. Photostable screening cosmetic composition according to Claim 1, characterised in that the 1,4-benzene [di(3-methylidene-10-camphosulphonic)] acid is partially or totally neutralised with an alkali metal hydroxide, ammonium hydroxide or an alkanolamine.

8. Photostable screening cosmetic composition according to any one of Claims 1 to 7, characterised in that it has a pH of between 4 and 9 and preferably between 5.5 and 8.

9. Photostable screening cosmetic composition according to any one of Claims 1 to 8, characterised in that it contains 0.1 to 10% by weight and preferably 0.5 to 5% by weight relative to the total weight of the composition, of 1,4-benzene[di(3-methylidene-10-camphosulphonic)] acid before partial or total neutralisation.

10. Photostable screening cosmetic composition according to any one of Claims 1 to 9, characterised in that it contains 0.1 to 15% by weight and preferably 0.5 to 10% by weight relative to the total weight of the composition, of the photostable or chemically photostabilised UV-A screening system.

11. Photostable screening cosmetic composition according to any one of Claims 1 to 10, characterised in that it is provided in the form of a cream or a milk and may be packaged as an aerosol.

12. Photostable screening cosmetic composition according to any one of Claims 1 to 11, characterised in that it contains a solvent for solubilising fat-soluble screening agents chosen from mineral, animal or vegetable oils and waxes, fatty acids, fatty acid esters, fatty alcohols and lower monoalcohols or polyols containing 2 to 6 carbon atoms.

13. Photostable screening cosmetic composition according to any one of Claims 1 to 12, characterised in that it in addition contains cosmetic adjuvants chosen from thickeners, demulcents, humectants, emulsifiers, preservatives, antifoams, lanolin, perfumes, propellants, colorants and pigments.

14. Photostable screening cosmetic composition intended for protecting the skin from UV radiation of wavelengths between 280 and 380 nm, in the form of a concentrate which can be diluted with water, or in an emulsion, characterised in that it contains, in a base consisting of a soap and water, a photostable screening agent with a broad absorption band consisting of 1,4-benzene [di(3-methylidene-10-camphosulphonic)] acid which is partially or totally neutralised, and a photostable system screening out UV-A radiation, comprising at least one of the following compounds: N-(2-ethylhexyl)-3-[(3′-methoxy-4′-n-butoxy)benzylidene]-10-camphosulphonamide (compound 2), 4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8),
it being possible for the total concentration of the screening agents defined above to be as high as 50% by weight.

15. Photostable screening cosmetic composition in the form of a concentrate according to Claim 14, characterised in that it contains 10 to 17% by weight of screening agent with a broad band, consisting of 1,4-benzene [di(3-methylidene-10-camphosulphonic)] acid (compound 1), and 15 to 33% by weight of screening agents consisting of the compounds 2 to 8 according to Claim 14.

16. Photostable screening cosmetic composition according to Claim 15, characterised in that it contains 10 to 17% by weight of screening agent consisting of the compound 1, up to 5.5% by weight of compound 3, up to 27% by weight of compound 6, and 12 to 15% by weight of a fatty acid soap, the rest consisting of water.

**Claims for the following Contracting State : ES**

1. Photostable screening cosmetic composition in the form of an emulsion intended for protecting the skin from UV radiation of wavelengths between 280 and 380 nm, characterised in that it contains in combination, in the aqueous phase, a photostable screening agent with a broad absorption band consisting of 1,4-benzene[di(3-methylidene-10-camphosulphonic)] acid which is partially or totally neutralised, and, in the oily phase, a photostable system screening out UV-A radiation, comprising at least one of the following compounds: N-(2-ethylhexyl)-3-[(3′-methoxy-4′-n-butoxy)benzylidene]-10-camphosulphonamide (compound 2), 4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8).

2. Photostable screening cosmetic composition according to Claim 1, characterised in that it contains, in the oily phase, N-(2-ethylhexyl)-3-[(3′-methoxy-4′-n-butoxy)benzylidene]-10-camphosulphonamide (compound 2) or 4-(1,1-dimethylethyl)-4′-methoxydibenzoylmethane (compound 3) which is photochemically stabilised by at least one compound chosen from benzylidenecamphor (compound 5), N-(2-ethylhexyl)-4-(3′-methylidenecamphor)benzenesulphonamide (compound 7) and 3-methoxy-4-n-butoxybenzylidenecamphor (compound 8) or a combination of the compounds 2 and 3 with one or more of the compounds 5, 7 and 8.

3. Photostable screening cosmetic composition according to Claim 1, characterised in that it contains, in the oily phase, 4-(1,1-dimethylethyl)-4′-methoxydibenzoylmethane (compound 3) which is photochemically stabilised by p-methylbenzylidenecamphor (compound 6).

4. Photostable screening cosmetic composition according to Claim 1, characterised in that it contains 4-(1,1-dimethylethyl)-4′-methoxydibenzoylmethane or 4-isopropyldibenzoylmethane, these compounds being photochemically stabilised by one or more benzylidenecamphor derivatives chosen from the compounds 5 to 8, the ratio by weight of the stabilising benzylidenecamphor derivative(s) to the dibenzoylmethane derivative(s) being not less than 1.

5. Photostable screening cosmetic composition according to Claim 4, characterised in that the ratio by weight

EP 0 365 370 B1

of the stabilising benzylidenecamphor derivative(s) to the dibenzoylmethane derivative(s) is not more than 6.

6. Photostable screening cosmetic composition according to any one of Claims 1 to 5, characterised in that it in addition contains homomenthyl salicylate in proportions ranging up to 6% by weight relative to the total weight of the composition.

7. Photostable screening cosmetic composition according to Claim 1, characterised in that the 1,4-benzene [di(3-methylidene-10-camphosulphonic)] acid is partially or totally neutralised with an alkali metal hydroxide, ammonium hydroxide or an alkanolamine.

8. Photostable screening cosmetic composition according to any one of Claims 1 to 7, characterised in that it has a pH of between 4 and 9 and preferably between 5.5 and 8.

9. Photostable screening cosmetic composition according to any one of Claims 1 to 8, characterised in that it contains 0.1 to 10% by weight and preferably 0.5 to 5% by weight relative to the total weight of the composition, of 1,4-benzene[di(3-methylidene-10-camphosulphonic)] acid before partial or total neutralisation.

10. Photostable screening cosmetic composition according to any one of Claims 1 to 9, characterised in that it contains 0.1 to 15% by weight and preferably 0.5 to 10% by weight relative to the total weight of the composition, of the photostable or chemically photostabilised UV-A screening system.

11. Photostable screening cosmetic composition according to any one of Claims 1 to 10, characterised in that it is provided in the form of a cream or a milk and may be packaged as an aerosol.

12. Photostable screening cosmetic composition according to any one of Claims 1 to 11, characterised in that it contains a solvent for solubilising fat-soluble screening agents chosen from mineral, animal or vegetable oils and waxes, fatty acids, fatty acid esters, fatty alcohols and lower monoalcohols or polyols containing 2 to 6 carbon atoms.

13. Photostable screening cosmetic composition according to any one of Claims 1 to 12, characterised in that it in addition contains cosmetic adjuvants chosen from thickeners, demulcents, humectants, emulsifiers, preservatives, antifoams, lanolin, perfumes, propellants, colorants and pigments.

14. Photostable screening cosmetic composition intended for protecting the skin from UV radiation of wavelengths between 280 and 380 nm, in the form of a concentrate which can be diluted with water, or in an emulsion, characterised in that it contains, in a base consisting of a soap and water, a photostable screening agent with a broad absorption band consisting of 1,4-benzene [di(3-methylidene-10-camphosulphonic)] acid which is partially or totally neutralised, and a photostable system screening out UV-A radiation, comprising at least one of the following compounds: N-(2-ethylhexyl)-3-[(3′-methoxy-4′-n-butoxy)benzylidene]-10-camphosulphonamide (compound 2),4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8),
it being possible for the total concentration of the screening agents defined above to be as high as 50% by weight.

15. Photostable screening cosmetic composition in the form of a concentrate according to Claim 14, characterised in that it contains 10 to 17% by weight of screening agent with a broad band, consisting of 1,4-benzene [di(3-methylidene-10-camphosulphonic)] acid (compound 1), and 15 to 33% by weight of screening agents consisting of the compounds 2 to 8 according to Claim 14.

16. Photostable screening cosmetic composition according to Claim 15, characterised in that it contains 10 to 17% by weight of screening agent consisting of the compound 1, up to 5.5% by weight of compound 3, up to 27% by weight of compound 6, and 12 to 15% by weight of a fatty acid soap, the rest consisting of water.

20

17. Process for preparing a photostable screening cosmetic composition in the form of an emulsion intended for protecting the skin from UV radiation of wavelengths between 280 and 380 nm, characterised in that it consists:

a) in a first stage, in dissolving in an aqueous phase 0.1 to 10% by weight of a photostable screening agent with a broad absorption band consisting of 1,4-benzene[di(3-methylidene-10-camphosulphonic)] acid, which is partially or totally neutralised with an alkali metal hydroxide, ammonium hydroxide or an alkanolamine, and in then adding the emulsifier;

b) in a second stage, in dissolving in a fatty phase, comprising mineral, animal or vegetable oils or waxes, fatty acids, fatty acid esters, fatty alcohols, lower monoalcohols or polyols containing 2 to 6 carbon atoms or a mixture thereof, 0.1 to 15% by weight of a photostable system screening out UV-A radiation comprising at least one of the following compounds: N-(2-ethylhexyl)-3-[(3′-methoxy-4′-n-butoxy)benzylidene]-10-camphosulphonamide (compound 2),4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8), the percentages of screening agents used in stages a) and b) being calculated on the basis of the total weight of the emulsion;

c) in a third stage, in heating the aqueous and fatty phases separately, at a temperature of about 75-80°C, and in adding the fatty phase to the aqueous phase with vigorous stirring.

18. Process for preparing a photostable screening cosmetic composition intended for protecting the skin from UV radiation of wavelengths between 280 and 380 nm, in the form of a concentrate which can be diluted with water, or in an emulsion, characterised in that it consists in dissolving in a base consisting of a soap and water 10 to 17% by weight of a photostable screening agent with a broad absorption band consisting of 1,4-benzene [di(3-methylidene-10-camphosulphonic)] acid and 15 to 33% by weight of a photostable system screening out UV-A radiation comprising at least one of the following compounds: N-(2-ethylhexyl)-3-[(3′-methoxy-4′-n-butoxy)-benzylidene]-10-camphosulphonamide (compound 2), 4-(1,1-dimethylethyl)-4′-methoxydibenzoylmethane (compound 3) and 4-isopropyldibenzoylmethane (compound 4), these last two compounds 3 and 4 being photochemically stabilised by at least one fat-soluble screening agent with a $\lambda_{max}$ of less than 330 nm which is chosen from the following benzylidenecamphor derivatives: benzylidenecamphor (compound 5), p-methylbenzylidenecamphor (compound 6), N-(2-ethylhexyl)-4-(3′-methylidenecamphor)benzenesulphonamide (compound 7) and 3-methoxy-4-n-butoxybenzylidenecamphor (compound 8), the percentages of screening agents used being calculated on the basis of the total weight of the concentrate.